# EUROPEAN PATENT SPECIFICATION

(11) **EP 0 579 038 B1**
(45) Date of publication and mention of the grant of the patent: **02.09.1998**
(21) Application number: 93110437.6
(22) Date of filing: 30.06.1993
(51) Int. Cl.: A61B 17/072

(54) **Apparatus for applying surgical fasteners**
Gerät zum Anbringen chirurgischer Befestigungselemente
Instrument pour poser les agrafes chirurgicales

(30) Priority: 30.06.1992 US 906766
(43) Date of publication of application: 19.01.1994
(73) Proprietor: United States Surgical Corporation, Norwalk, Connecticut 06856 (US)
(72) Inventor: Green, David T., Westport, CT 06880 (US); Ehrenfels, Karl H., Danbury, CT 06810 (US); Kappel, Gary S., Stamford, CT 06906 (US)
(74) Representative: Marsh, Roy David

(56) References cited:
- EP-A- 0 479 131
- FR-A- 2 542 188
- US-A- 3 269 630
- US-A- 4 442 964
- US-A- 4 527 724
- US-A- 4 530 453
- US-A- 4 606 344

## Description

The present invention relates to apparatus for applying surgical fasteners to body tissue, comprising:
a frame portion;
a handle mechanism depending from said frame portion and coupled to means for driving said fasteners into tissue responsive to movement of said handle mechanism;
tissue gripping means including a first jaw member having a plurality of fasteners positioned thereon, and a second jaw member, said tissue gripping means positioned at one end of said frame portion;
means operable independently of the handle mechanism for advancing one of said jaw members towards said other jaw member to selectively position said one jaw member; and
means for preventing driving of said fasteners.

Such apparatus is described in US-A-4 354 628 and US-A-4 530 453.

Surgical fastening devices having means for controlling the spacing between the jaw members are well known in the art. These devices typically include indicating means to provide a reading of the spacing between the jaw members. Devices are also known in the art which provide latching mechanisms to actuate the firing mechanism only when the distance between the jaws is within a preset range. These devices typically include a complex lock-out mechanism.

Various closing mechanisms are provided in the prior art for use with surgical fastening devices. The most notable of these devices utilize a complex worm gear-type arrangement or screw bearing member to open and close the spacing between the jaw members of the surgical fastening apparatus. These devices generally provide a rotatable knob or wing-like assembly at the trigger end of the device remote from the jaw mechanism which carries the fastener cartridge, and a screw-like mechanism is provided that passes through the body of the device to translate the rotational movement of the knob into longitudinal movement of the cartridge frame to open and close the spacing between the jaws. As the jaw members are closed around a tissue site to which fasteners are to be applied, the surgeon must grasp the device with one hand while rotating the knob or wing-like assembly with the other hand. As the jaws members close about the tissue to pinch the tissue therebetween, the surgeon then ceases rotation and activates the trigger mechanism to drive the fasteners into the tissue. Several known devices provide a trigger-like mechanism, while others provide a secondary rotatable knob for driving the fasteners by rotational movement. Many devices provide an indicator means near the rotatable knob which gives a visual indication of the spacing between the jaw members prior to firing.

These prior art devices are subject to several disadvantages in both use and construction which render these devices difficult to operate and expensive to manufacture. Many of the devices are cumbersome in use in that the surgeon must operate the device with both hands, holding the body of the instrument in one hand while rotating the knob or wing assembly with the other hand. This may lead to inaccurate stapling or fastening since the surgeon is unable to guide the tissue to be stapled or fastened with his free hand while closing the jaws about the tissue. Furthermore, the number of interacting components provides inaccuracies due to normal break down of tolerances. In addition, the gear arrangement may become worn during extended use, thus rendering an imprecise grasping action at the jaws.

Furthermore, these prior art devices generally involve a complex construction in which a precisely machined or cast worm gear must be constructed and incorporated into the device. This of course increases the cost of manufacturing, and requires a sophisticated assembly procedure to properly locate the worm gear in the instrument to control the spacing between the jaws.

Typical devices having a rotatable knob at the end portion adjacent the handle mechanism of the surgical stapling or fastening device are disclosed in, among others, U.S. Patent No. 4,930,503 to Pruitt, U.S. Patent No. 4,788,978 to Strekopytov et al., and U.S. Patent No. 4,606,344 to DiGiovanni. In each of these devices, an elongated rod member having screw threads machined thereon is provided, which connects a rotatable knob positioned adjacent the handle members to a pusher mechanism which urges a movable jaw in a forward direction toward a stationary jaw to close the spacing between the jaw members. When a desired spacing is reached, a trigger mechanism may be activated to fire the fasteners through the tissue into the anvil member mounted on the stationary jaw. To remove the fastening instrument after application of the fasteners, the knob is rotated in an opposite direction which turns the screw threaded rod member to move the movable jaw member away from the stationary jaw member so that the entire device maybe removed from the tissue.

Surgical fastening instruments having a wing like arrangement positioned adjacent the handle assembly of a device for moving a movable jaw toward a stationary jaws for affixing surgical fasteners to tissue are disclosed in U.S. Patent No. 4,442,964 to Becht, U.S. Patent No. 4,354,628 to Green, and U.S. Patent No. 3,795,034 to Strekopytov et al. These devices are similar to those described above except for the provision of a rotatable wing member in place of the rotatable knob. These devices are also provided with a screw threaded rod member which, when rotated, urges a movable jaw towards a stationary jaw to close the jaw members around tissue to be fastened together. After the application of surgical fasteners, the wing assembly is rotated in an opposite direction to draw the movable jaw away from the stationary jaw so that the instrument maybe removed from the tissue.

Surgical stapling or fastening instruments having a pivotable mechanism external to the device for moving a movable jaw toward a stationary jaw prior to affixing surgical fasteners to tissue are disclosed in, among others, U.S. Patent No. 3,269,630 to Fleischer, U.S. Patent No. 4,530,453 to Green, U.S. Patent No. 4,715,520 to Roehr, Jr. et al., and U.S. Patent No. 4,978,049 to Green.

Green ('453), Roehr, Jr. et al. and Green ('049) each disclose a pivotable lever member which urges a movable jaw into proximity of a stationary jaw prior to application of the surgical fasteners. Fleischer discloses a surgical stapling instrument in which a pivotable handle urges the movable staple cartridge against the tissue in the direction of the stationary jaw and fires the staples in the same motion. In each of these devices, removal of the instrument after firing of the surgical fasteners is accomplished by pivoting the lever mechanism in the opposite direction to open the jaw members by moving the movable jaw away from the stationary jaw.

US-A-5 190 203 discloses a spring biased pivotal catch member for approximating the jaws which is held in selected position by a pointed lance member.

It would be desirable for a surgical fastening device to include a self-deactivating locking structure for preventing undesirable firing of fasteners before an appropriate distance between a fasteners cartridge and an anvil has been achieved.

According to the invention, the apparatus initially defined is characterized by: said preventing means being self-deactivating when said first jaw member is positioned a predetermined distance from said second jaw member; and said preventing means being positioned within said frame portion.

The foregoing features of the present invention will become more readily apparent and may be understood by referring to the following detailed description of an illustrative embodiment of the surgical fastening instrument and its novel adjustable closure mechanism, taken in conjunction with the accompanying drawings, in which:
Fig. 1 illustrates a perspective view of a surgical fastening instrument employing an adjustable closure mechanism;
Fig. 2 illustrates a side cross-sectional plan view of the surgical fastening instrument of Fig. 1 with the instrument in an at rest condition;
Fig. 3 illustrates the device of Fig. 2 in which the adjustable closure mechanism is activated and the jaw mechanism is partially closed;
Fig. 4 illustrates the device of Fig. 2 in which the adjustable closure mechanism is fully deployed;
Fig. 5 illustrates the device of Fig. 2 in which the adjustable closure mechanism is fully deployed and the trigger mechanism of the device has been actuated so that the fastening means have been driven from the cartridge;
Fig. 6 illustrates a partial enlarged view of the handle end of the device of Fig. 2 showing the release mechanism for disengaging the retaining means;
Fig. 7 illustrates the retaining means at the handle end of the device of Fig. 2 in the at rest condition;
Fig. 8 illustrates a top plan cutaway view of the instrument of Fig. 1 showing the adjustable closure mechanism in the at rest condition;
Fig. 9 shows a top plan cutaway view of the instrument of Fig. 1 showing the adjustable closure mechanism in the fully deployed condition;
Fig. 10 illustrates a perspective view of surgical fastening apparatus employing an alternative embodiment of the adjustable closure mechanism;
Fig. 11 illustrates a side cutaway view of the handle end of the instrument of Fig. 10 showing the adjustable closure mechanism;
Fig. 12 illustrates a top plan cutaway view of the device of Fig. 10 showing the linkage arrangement of the adjustable closure mechanism in an at rest condition;
Fig. 13 illustrates a top plan cutaway view of the device of Fig. 10 showing the linkage arrangement of the adjustable closure mechanism in a fully deployed condition;
Figs. 14A-14C illustrate a coupling mechanism according to the present invention for coupling the trigger mechanism to the fastener driving mechanism used in conjunction with the adjustable closure mechanism;
Figs. 15A and 15B illustrate embodiments of the retaining means of the adjustable closure mechanism;
Fig. 16 illustrates a side cutaway view of an instrument showing a locking structure according to the present invention; and
Figs. 17-19 illustrate side cutaway views of the instrument shown in Fig. 16 showing a sequence of operation of the instrument.

The following describes a surgical fastening device having a mechanism for adjusting the distance between the movable jaw and the stationary jaw prior to the application of fasteners to the body tissue. The adjustable mechanism controls the closing of the jaw mechanism to approximate the distance between the jaw members prior to activation of the trigger mechanism to fire the fasteners. The device may be operated with one hand, which frees the surgeon to accurately locate the tissue to be repaired and to place the fasteners in the proper position during the procedure. The adjustable closure mechanism is operable by using the thumb of the hand which holds the device, and linearly moves the stapling mechanism to properly approximate the distance between the jaw members. The adjustable closure mechanism eliminates many moving parts associated with prior devices, and provides a device which is lightweight, and easy to use by allowing the surgeon to set and release the device with one hand.

The adjustable closure mechanism described hereinafter may be used with any surgical instrument having jaw members which include a stationary jaw and a movable jaw, or two movable jaws, in which the spacing between the jaw members is adjustable to accommodate various thicknesses of tissue to be secured. The provision of a push button at the handle end of the instrument and the elimination of numerous complex moving parts which are common in prior art devices allows the surgeon to approximate the distance between the jaw members in a fast and efficient manner to position the jaws in the proper alignment for the application of surgical fasteners.

The apparatus comprises a first jaw member and a second jaw member in which the first jaw member includes a plurality of fasteners positioned in a cartridge which is movable with the first jaw member towards the stationary second jaw member. The second jaw member may include an anvil surface for clinching the fasteners, or may include means for engaging the fasteners to secure the tissue therebetween. Means for advancing the first jaw member towards the second jaw member to grip the tissue between the jaws are provided, as well as releasable means for retaining the advancing means along a linear path of travel to selectively position the first jaw member in relation to the second jaw member. Means for driving the fasteners into the tissue subsequent to positioning the jaws members in relation to each other by the advancement means is also provided, and the advancement means of the apparatus is independent of the driving means.

In a preferred embodiment a push button mechanism is provided at the handle end of the device which may be linearly displaced by the thumb of the surgeon. As the push button and slider bar arrangement is urged forwardly towards the jaws, the releasable retaining means is also urged forwardly within the housing of the apparatus to selectively position the jaw members in relation to each other. As the slider bar and releasable retaining means are continuously moved forward, a linkage arrangement is activated which urges the cartridge frame forward so that the cartridge moves towards the anvil. When the linkage arrangement is fully actuated, the proper distance between the jaw members is set, so that the trigger mechanism may be actuated to drive the fasteners through the tissues.

According to the present invention, a coupling mechanism is provided which couples the fastener driving means to the trigger mechanism to allow for driving of the staples or fasteners when the proper distance between the jaw members is set. As the slider mechanism is moved forward and the linkage arrangement actuated, the fastener driving means is urged forwardly with the cartridge frame. A coupling arm, which is connected at one end to the trigger mechanism, slides along a bearing surface on the driving means until the slider mechanism is fully deployed. At this point, a camming edge of the coupling arm engages a notch in the bearing surface of the driving means to couple the trigger mechanism to the driving means. At this point, the proper distance between the jaw members is set and the fastener means may be driven into the tissue.

The surgical fastening device may further include a locking structure for retaining a fastener driving structure in a predeterminable position. The locking structure is positioned proximate an actuating handle for selectively preventing the handle from actuating the driving structure. The driving structure cannot engage the fasteners until a first jaw member is positioned a specified distance from a second jaw member.

After the fastening means have been driven into the tissue, the releasable retaining mechanism may be disengaged so that the jaw members may be returned to their original position whereby the fastening device may be removed from the surgical site. In the preferred embodiment, the push button is pivotable to move a second rod member which contacts a release lever which disengages the retaining means. In a second embodiment, a release knob is provided which extends through the housing of the fastening apparatus and which may be pivoted to release the retaining means.

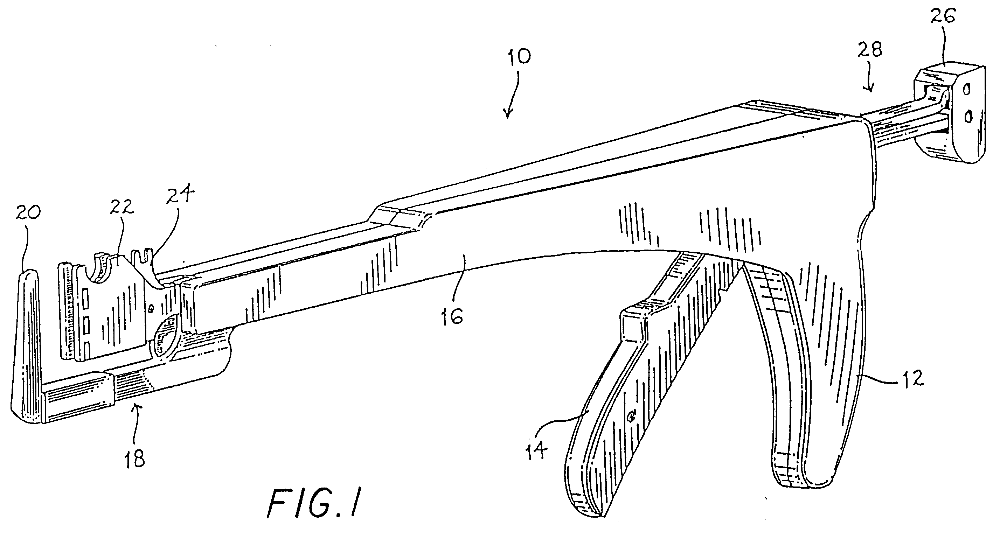

Referring now in specific detail to the drawings, in which like reference numerals identify similar or identical elements throughout the several views, Fig. 1 shows a surgical fastening instrument 10 which employs an adjustable closure mechanism. Fastening instrument 10 is provided with a stationary handle 12 and an actuating handle 14 which together comprise the trigger mechanism of instrument 10. An elongated body portion 16 is provided which terminates in a distal jaw mechanism 18 which includes an anvil jaw 20 and a artridge jaw 22. A fastening cartridge (not shown) is positioned within cartridge jaw 22 for driving staples or fasteners through tissue against an anvil surface positioned on anvil jaw 20. Alternatively, the cartridge can contain the fastener portions of two part fasteners which are driven into retainers positioned on the anvil jaw. At the handle end of instrument 10 is provided a push button 26 for operating an advancement mechanism 28, whose function will be described below.

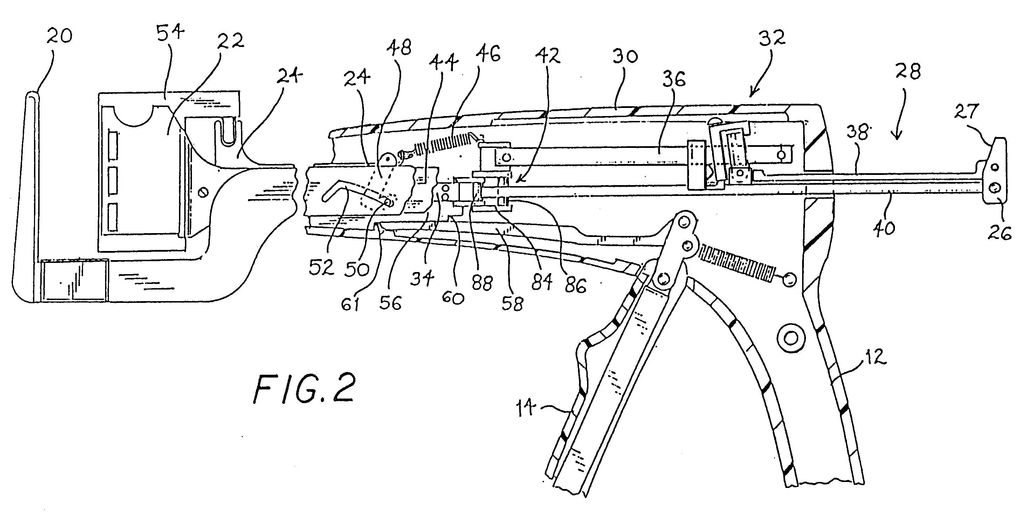

As seen in Fig. 2, push button 26 and advancing mechanism 28 extend outwardly from the handle end of the instrument 10. A releasable retaining mechanism 32 is slidably engaged to the stationary rod member 36 and is coupled to slider mechanism 40 so that as slider mechanism 40 is urged forwardly into housing 30, retaining mechanism 32 is slidably retained along stationary rod member 36.

Advancing mechanism 28 comprises slider mechanism 40 and release rod member 38, such that release rod member 38 and slider mechanism 40 are secured to push button 26. Thrusting push button 26 towards housing 30 slides release rod 38 and slider mechanism 40 into the housing to move the retaining mechanism 32 along rod 36. Slider mechanism 40 extends to linkage structure 42 to activate the linkage structure 42 and urge jaw mechanism 18 distally. Linkage structure 42 moves movable rod 34, as well as fastener driver 56, cartridge frame 44, alignment pin advancement means 24, and cartridge 54 all in a distal direction to selectively position movable cartridge jaw 22 and stationary anvil jaw 20.

For purposes of clarity, the individual mechanisms will be described separately, and then the overall operation of the device will be discussed.

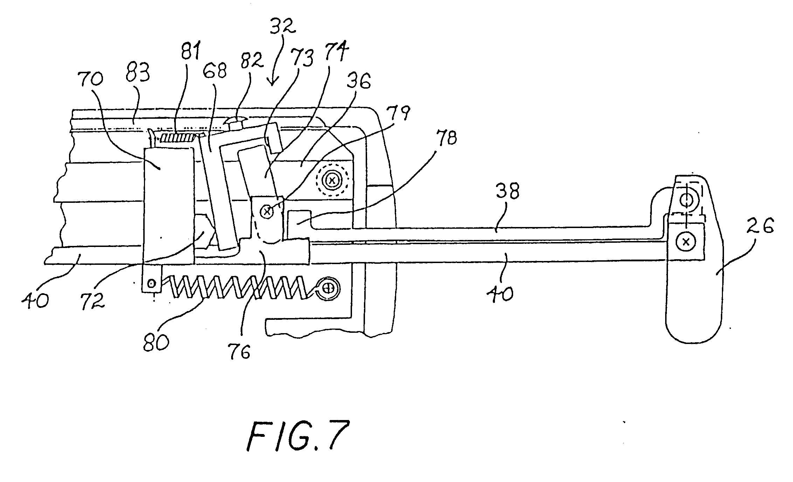

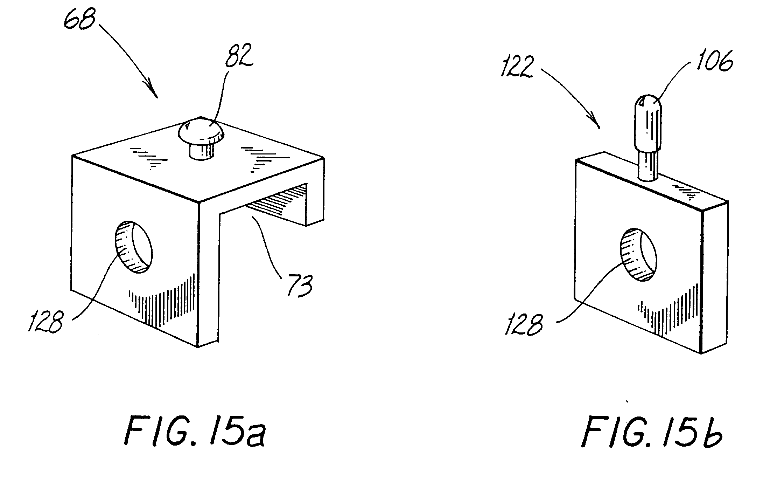

Fig. 7 illustrates a retaining mechanism which slidably engages the stationary rod 36, and which acts in conjunction with the linkage structure 42 to selectively position the jaw mechanism 18 of the surgical fastener apparatus 10. Retaining mechanism 32 is coupled to slider mechanism 40 and is urged rearwardly by biasing spring 80 as shown. The retaining mechanism 32 essentially comprises a clamp member 68 which is provided with a central bore 128 through which stationary rod 36 passes. Clamp member 68 is best seen in Fig. 15A. Retaining mechanism 32 further comprises a block member 70 to which clamp member 68 is pivotally secured and biased into a locking engagement of stationary rod 36 by spring member 81. Spring member 81 may comprise a coiled spring as shown, or may further comprise any other biasing mechanism such as a leaf spring, rubber block, or the like. Block member 70 may be provided with a central bore (not shown) through which stationary rod 36 passes, or alternatively, block member 70 may have a substantially U-shaped portion to allow stationary rod 36 to pass therethrough. Block member 70 further comprises shoulder portion 72 which abuts the lower portion of clamp member 68 as shown to provide a pivot point for releasing clamp member 68, as will be described below.

As best seen in Fig. 15A, clamp member 68 has an L-shaped portion terminating in a contact face 73 which engages a release lever 74, which is pivotably connected to carriage 76 and which pivots about pivot point 79. Release lever 74 preferably has a central bore to allow a stationary rod 36 to pass therethrough, but may also be provided with a U-shaped body both to surround stationary rod 36 and engage contact face 73 of clamp member 68. Clamp member 68 is further provided with a guide post 82 which slides within a guide track 83 to fully align clamp member 68 in relation to stationary rod 36.

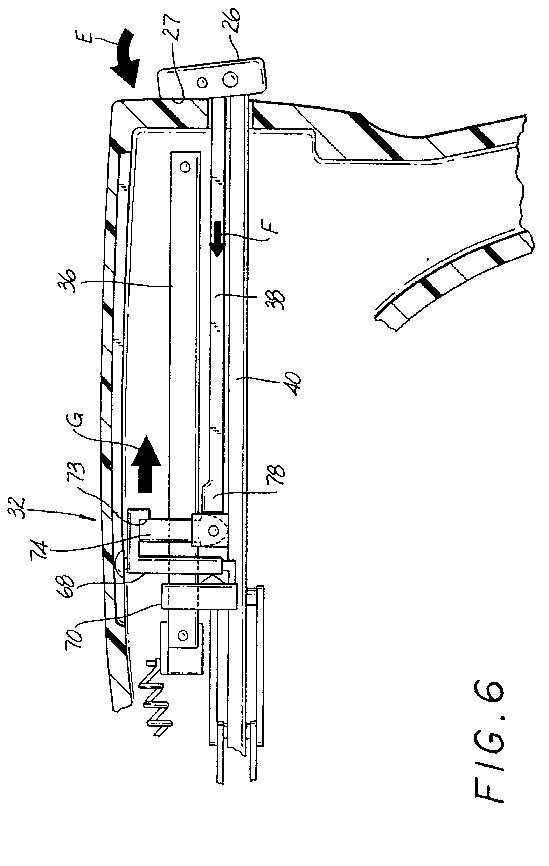

As shown in Fig. 7, clamp member 68 is biased at an angle to engage stationary rod 36 so that edges of central bore 128 frictionally engage stationary rod member 36. As push button 26 is urged towards housing 30, retaining mechanism 32 slides along stationary rod 36 due to the movement of advancing mechanism 28. Carriage 76 is engaged with movable slider mechanism 40 so that the entire retaining mechanism is urged distally against biasing spring 80. In order to release retaining mechanism 32, as best shown in Fig. 6, push button 26 is rotated in the direction of arrow E until beveled surface 27 contacts housing 30. Pivoting push button 26 in the direction of arrow E moves release rod 38 in the direction of arrow F so that contact surface 78 of release rod 38 pivots release lever 74 to engage contact face 73 of clamp member 68. This pivoting action moves clamp member 68 in the direction of arrow G to release the frictional engagement of the central bore 128 with stationary rod 36. Releasing the frictional engagement causes the entire retaining mechanism 32 to return in the direction of arrow G to the position shown in Fig. 7. This movement is caused by biasing spring 80 (not shown in Fig. 6) which moves the entire mechanism to the position shown in Fig. 7.

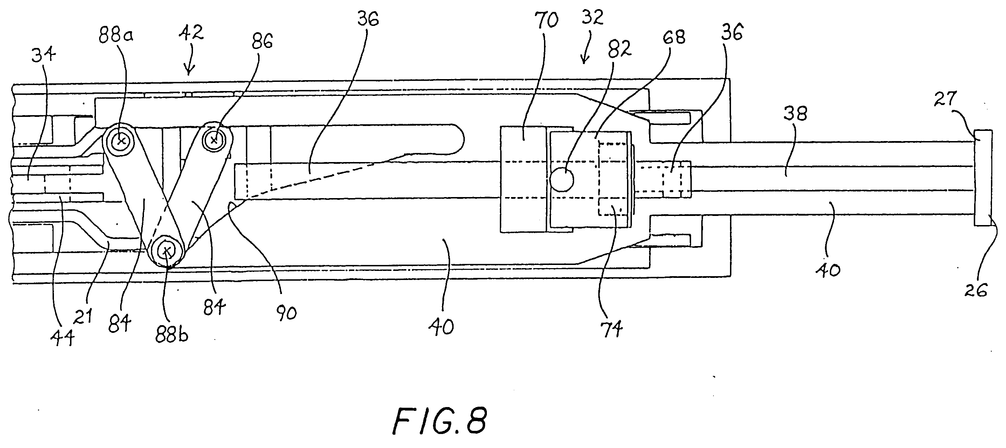

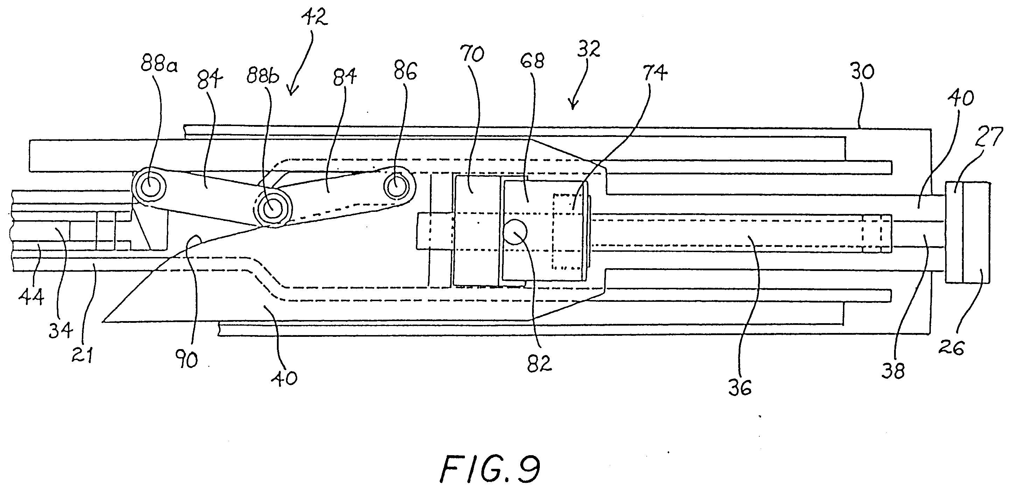

Turning now to Figs. 8 and 9, there is illustrated the linkage structure 42 and its operation in conjunction with slider mechanism 40 and retaining mechanism 32. Structure 42 comprises a pair of linkage arms 84, which are preferably secured by pivot posts to a second pair of linkage arms 84 located below the pair shown in Fig. 8 in mirror arrangement, as clearly shown in Figs. 2-6. Linkage arms 84 are joined through stationary pivot post 86, and movable pivot posts 88A and 88B. Movable pivot post 88A is secured to rod 34 and cartridge frame 44 to urge these elements distally when push button 26 is activated. Slider mechanism 40 includes a camming surface 90 which engages movable pivot post 88B to collapse linkage structure 42 to move the rod 34 and cartridge frame 44, and consequently move cartridge jaw 22 towards anvil jaw 20.

As best seen in Fig. 9, as push button 26 is fully actuated to contact housing 30, retaining mechanism 32, being coupled to slider mechanism 40 slides along stationary rod 36. Camming surface 90 engages movable post 88B, driving movable post 88A distally to move movable rod 34 and cartridge frame 44 in relation to housing frame 21 as shown. Releasing retaining mechanism 32 as described above returns linkage structure 42 to the configuration shown in Fig. 8.

It can be appreciated from Figs. 8 and 9 that the linkage structure 42 provides a two-stage approximation of the jaw mechanism 18, whereby initial movement of the slider mechanism 40 caused a large initial approximation, while a smaller, secondary approximation eases the jaws into approximation at the conclusion of movement of the slider mechanism 40. As slider mechanism 40 is initially moved upon actuation of the push button 26, a large portion of the overall distance cartridge jaw member 22 travels towards anvil jaw member 20 is traversed in the initial movement. Typically, as the slider mechanism 40 travels approximately one-half its overall distance, and correspondingly moving movable pivot post 88A a portion of its total distance, cartridge jaw 22 moves approximately 80% of its total distance. As slider mechanism 40 travels its remaining one-half distance, the cartridge jaw moves its final 20% of its total distance. This allows for a fine adjustment of the jaw mechanism to accommodate the various thickness of tissues positioned between the jaw members.

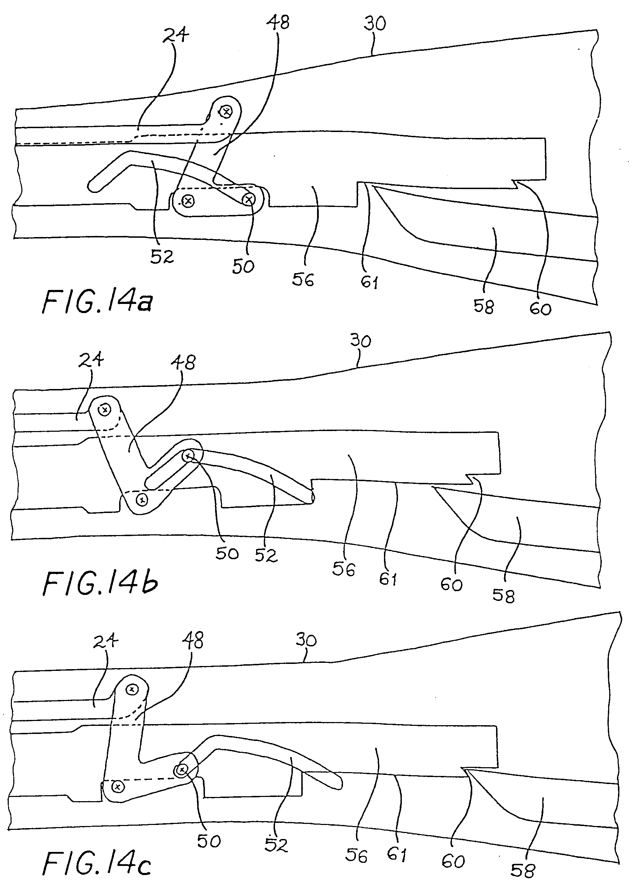

Instrument 10 employing the adjustable closure mechanism further includes a coupling device for coupling the fastener driving mechanism to the trigger mechanism only when a proper distance between cartridge jaw 22 and anvil jaw 20 has been reached. This mechanism is best illustrated in Figs. 14A through 14C.

Figs. 14A through 14C, in conjunction with Figs. 2-6 illustrate the coupling mechanism.

Housing frame 21 is provided with a frame track 52 within which a driving pin 50 rides. Driving pin 50 is secured to one leg of an L-shaped driving link 48, where the opposite end of driving link 48 is secured to the alignment pin advancement means 24. Driving link 48 is further coupled to cartridge frame 22 which is advanced distally when push button 26 is actuated. As push button 26 is actuated, linkage structure 42 is deployed and fastener driver 56 is moved distally. Prior to actuation of push button 26, fastener driver 56 is in the position shown in Fig. 14A, and coupling arm 58 is positioned on bearing surface 61 as shown. Coupling arm 58 is connected to actuating handle 14 as best seem in Fig. 2.

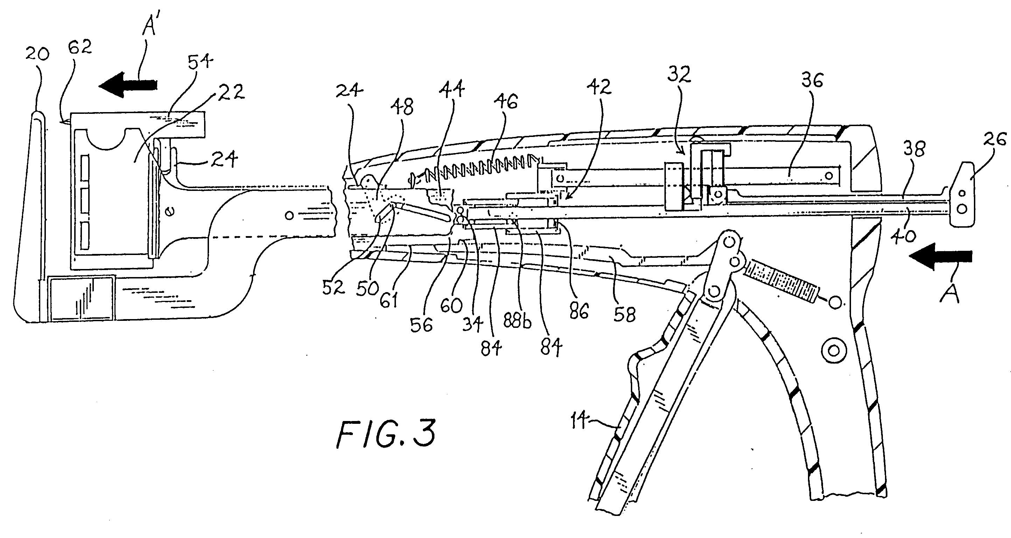

As push button 26 is moved, fastener driver 56 is moved forwardly so that coupling arm 58 slides along bearing surface 61 as shown in Fig. 14B. Driving pin 50 travels in frame track 52, while driving link 48 urges alignment pin advancement means 24 as shown. As best seen in Fig. 3, alignment pin advancement means 24 moves forwardly so that alignment pin 62 protrudes from cartridge 54 and aligns with an alignment hole (not shown) in anvil jaw 20. This insures proper alignment of cartridge 54 with anvil jaw 20 so that fastener means 66 are properly driven into position between the jaw members.

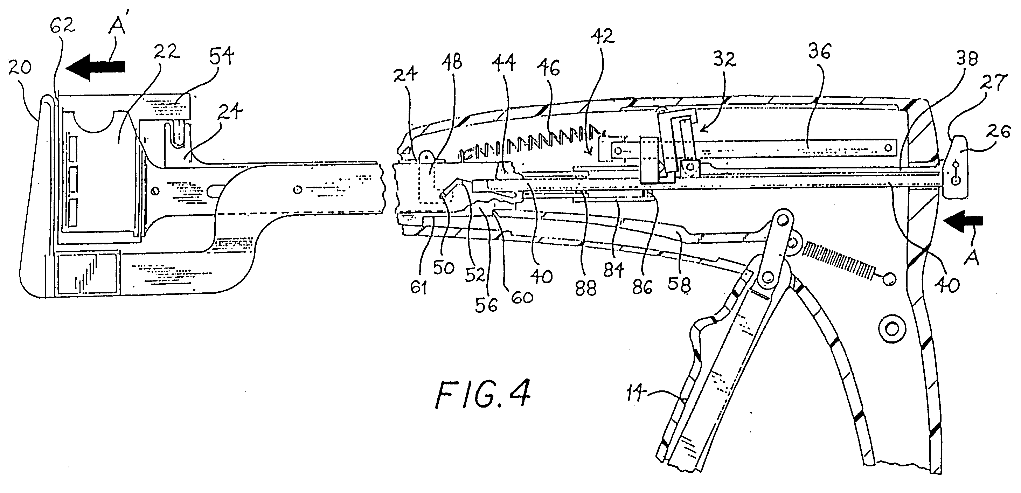

As push button 26 is further moved towards housing 30, to the position shown in Fig. 4, cartridge jaw 22 is aligned adjacent anvil jaw 20 so that pin 62 is within the hole in anvil jaw 20. Driving link 48 moves slightly in the proximal direction towards the handle end of instrument 10 to a substantially upright position as shown in Fig. 14C and Fig. 4. This moves alignment pin advancement means 24 slightly proximally to the position shown in Fig. 4 so that alignment pin 62 does not protrude completely through anvil jaw 20.

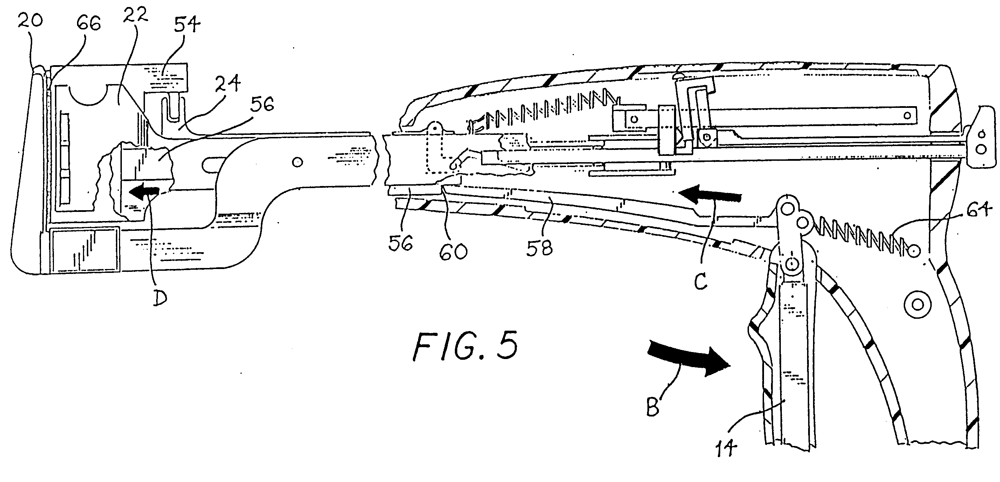

When push button 26 reaches the position shown in Fig. 4, fastener driver 56 has moved distally to a position where coupling arm 58 slides off bearing surface 61 and into notch 60 as shown in Fig. 14C. At this point, driving link 48 has moved to the position shown in Fig. 14C and driving pin 50 has fully traversed the length of frame track 52. In the position shown in Fig. 14C, coupling arm 58 is engaged with fastener driver 56 so that actuation of handle 14 as shown in Fig. 5 will drive fastener means 66 into the tissue as fastener driver 56 moves in the direction of arrow D. Although not shown, coupling arm 58 may be provided with a leaf spring member to urge coupling arm 58 into engagement with notch 60. As push button 26 is rotated to release retaining mechanism 32, driving pin 50 travels proximally in frame track 52, so that when driving pin 50 reaches the position shown in Fig. 14B fastener driver 56 is lifted off coupling arm 58 despite the leaf spring, and coupling arm 58 is no longer engaged in notch 60. As retaining means 32 returns the entire mechanism to the position shown in Fig. 2, driving link 48 and fastener driver 56 return to the position shown in Fig. 14A.

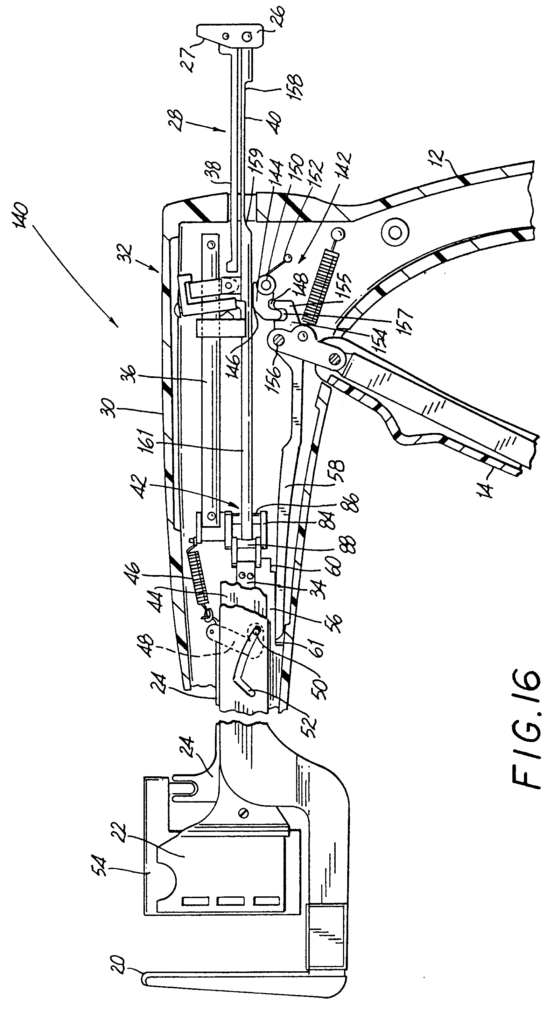

Referring to Fig. 16, another embodiment of a surgical fastening instrument 140 is shown having a locking structure 142. The surgical fastening instrument 140 shown in Fig. 16 is substantially similar to the surgical fastening instrument 10 shown in Figs. 1-7. However, the locking structure 142 of the surgical fastening instrument 140 provides positive locking of the actuating handle 14 during a specified period of the operation of the instrument 140.

The locking structure 142 includes a cam member 144. The cam member 144 includes an arcuate portion 146 having a recess 148. A proximal end of the cam member 144 is pivotally connected to pivot point 150. The cam member 144 is biased in an upward position by spring 152.

The locking structure further includes a hook member 154 which is formed in the proximal end of the coupling arm 58. The hook member 154 includes an arcuate portion 155 defining a notch 157. The notch 157 of the hook member 154 is configured and dimensioned to releasably couple with the arcuate portion 146 of the cam member 144.

Cam member 144 is pivotally mounted within handle 12 in a position below slide mechanism 40. An elongated longitudinal camming groove 158 is formed adjacent the proximal end of the lower portion of slide mechanism 40. The groove 158 has a ramped camming surface 159 at a distal end thereof. Groove 158 is dimensioned to provide a plurality of positions between the anvil jaw 20 and the cartridge jaw 22 in which the instrument can be fired (described below). Camming surface 159 defines the proximalmost position of cartridge jaw 22 where the handle 14 can be squeezed and the fasteners fired.

Returning now to Figs. 2 through 6, the operation of the surgical fastener apparatus 10 having the adjustable closure mechanism will now be described.

After tissue which is to be surgically repaired is positioned between cartridge jaw 22 and anvil jaw 20, push button 26 is pushed in the direction of arrow A as seen in Fig. 3 which moves slider mechanism 40 and release rod 38 into housing 30. Retaining mechanism 32 is slid distally along stationary rod 36, and camming surface 90 of slider mechanism 40 engages stationary post 88B to deploy linkage structure 42. As linkage structure 42 is deployed, movable rod 34 is urged forwardly along with cartridge frame 44, thus urging driving pin 50 along frame track 52. The force of biasing spring 46 is overcome as push button 26 is urged in the direction of arrow A.

As driving pin 50 moves in track 52, driving link 48 is moved to the position shown in Fig. 3, which urges alignment pin advancement means 24 to the position shown at the jaw mechanism 18. In this position, alignment pin 62 protrudes from cartridge 54 and aligns with the alignment hole in anvil jaw 20 as cartridge 54 moves in the direction of arrow A'.

As linkage structure 42 is deployed and movable rod 34 and cartridge frame 44 move distally, fastener driver 56 also moves distally and coupling arm 58 slides along bearing surface 61.

When push button 26 is fully actuated, linkage structure 42 is fully deployed as shown in Fig. 4, and retaining mechanism 32 frictionally engages stationary rod 36 to maintain instrument 10 in the position shown in Fig. 4. At this time, cartridge 54 has moved into position in the direction of arrow A' so that alignment pin 62 is positioned in the alignment hole in anvil jaw 20. Alignment pin advancement means 24 moves slightly proximally so that alignment pin 62 does not protrude beyond anvil jaw 20, and driving link 48 assumes the position shown in Fig. 4. Driving pin 50 has reached the end of track 52. In the position shown in Fig. 4, actuating arm 58 has slid off bearing surface 61 and into notch 60 of fastener driver 56 so that the device as shown in Fig. 4 is ready to be fired.

Once in the position of Fig. 4, actuating handle 14 is moved in the direction of arrow B to fire the fasteners 66. As actuating handle 14 is moved in the direction of arrow B against the force of biasing spring 64, coupling arm 58, having been engaged in notch 60, moves in the direction of arrow C to move fastener driver 56 distally in the direction of arrow D. Fastener driver 56 drives fasteners 66 from cartridge 54 through the tissue (not shown) and into the anvil surface of anvil jaw 20. Upon completion of firing, actuating handle 14 is released and returns to the position shown in Fig. 4.

To remove instrument 10 from the surgical site, it is necessary to release the jaw mechanism 18 to return to the position shown in Fig. 2. This is accomplished by pivoting push button 26 in the direction of arrow E, as best seen in Fig. 6, so that beveled surface 27 contacts the housing 30. As push button 26 is pivoted in the direction of arrow E, release rod 38 travels in the direction of arrow F so that contact surface 78 of release rod 38 pivots release lever 74 as shown, which engages contact face 73 to move clamp member 68 to an upright position and perpendicular in relation to stationary rod 36. This releases the frictional engagement of clamp member 68 with stationary rod 36 and the entire retaining mechanism 32 is moved along stationary rod 36 in the direction of arrow G due to the force of biasing spring 80 (as shown in Fig. 7). The entire mechanism, including the linkage structure 42, jaw mechanism 18, and retaining mechanism 32 is returned to the position shown in Fig. 2.

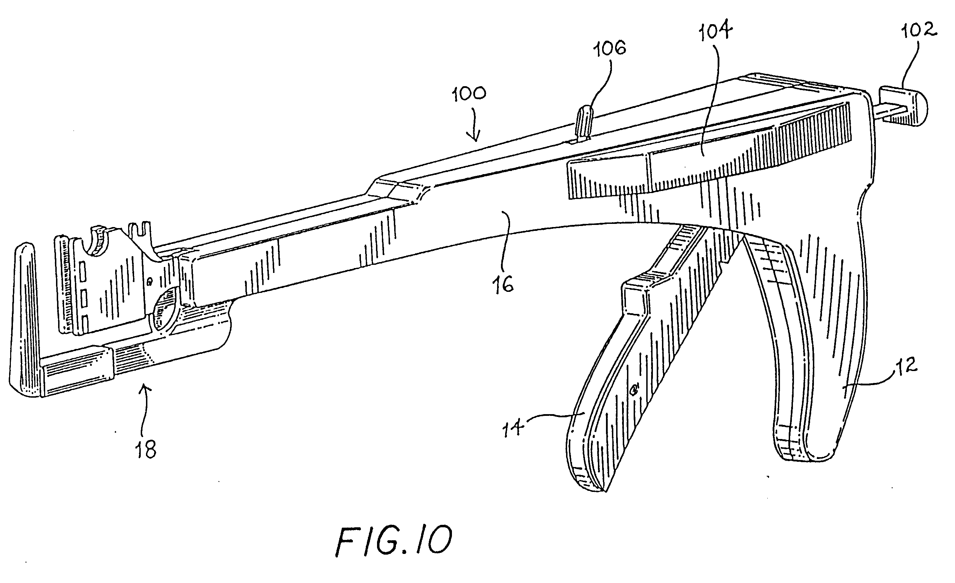

Fig. 10 illustrates a surgical fastening apparatus 100 employing an alternative adjustable closure mechanism.

Apparatus 10 is similar to apparatus 10 of Fig. 1 in that a stationary handle 12 and an actuating handle 14 are provided, along with a body portion 16 and a jaw mechanism 18. Body portion 16 is provided with a flared portion 104 which is symmetrical on both sides of the instrument for accommodating the slider mechanism which will be described below. A push button 102 is provided for actuating the slider mechanism, and a release button 106 is provided to release the retaining mechanism as will be described below.

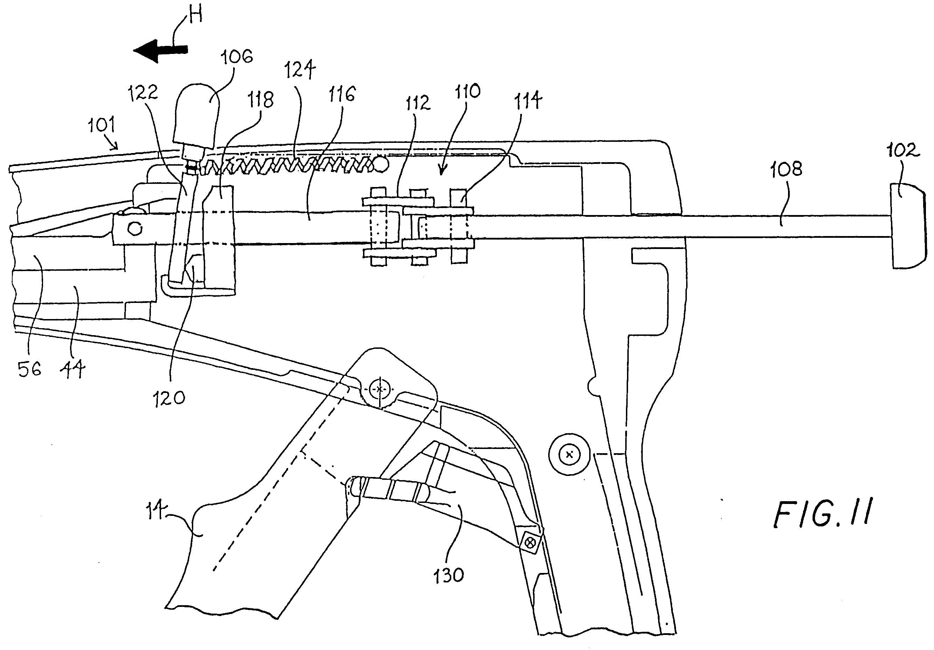

Turning now to Fig. 11, there is shown the adjustable closure mechanism of the apparatus of Figure 10. Instrument 100 is substantially identical to instrument 10 except for retaining mechanism 101 and linkage structure 110.

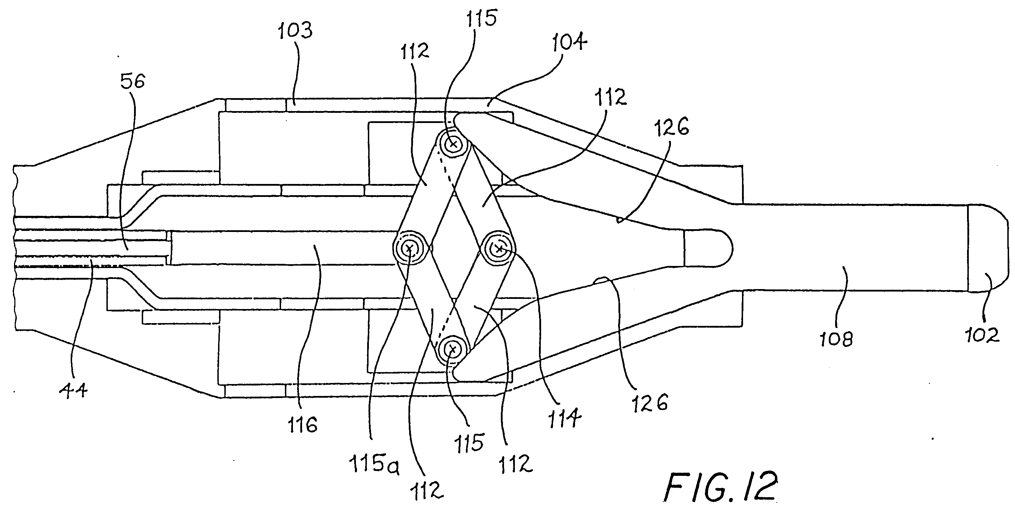

Linkage structure 110 comprise a plurality of linkage arms 112, as best seen in Fig. 12. Linkage arms 112 form a collapsible box structure having a mirror image as shown in Fig. 11. Linkage arms 112 are joined by stationary pivot post 114 and movable pivot posts 115. As seen in Fig. 12, movable pivot post 115A is secured to movable rod 116 whose function will be described below. Push button 102 is connected to slider mechanism 108 which is provided with an essentially Y-shaped configuration. The outer ends of the Y-shaped slider mechanism are accommodated in flared portions 104 of the housing 103 of instrument 100. Movable rod 116 extends from movable pivot point 115A through retaining mechanism 101 to connect to fastener driver 56 and cartridge frame 44 as shown. Movable rod 116 is frictionally engaged by retaining mechanism 101 to selectively position cartridge jaw 22 in relation to anvil jaw 20.

Retaining mechanism 101 comprises clamp member 122 and block member 118 which is provided with shoulder 120. Clamp member 122, as best seem in Fig. 15B, is provided with a central bore 128 whose edges frictionally engage movable rod 116. Movable rod 116, as well as stationary rod 36 of the embodiment of Figs. 1-9, may be provided with a scored surface to enhance the frictional gripping of clamp members 122 and 68. Clamp member 122 is biased into the engaged position by biasing spring 124.

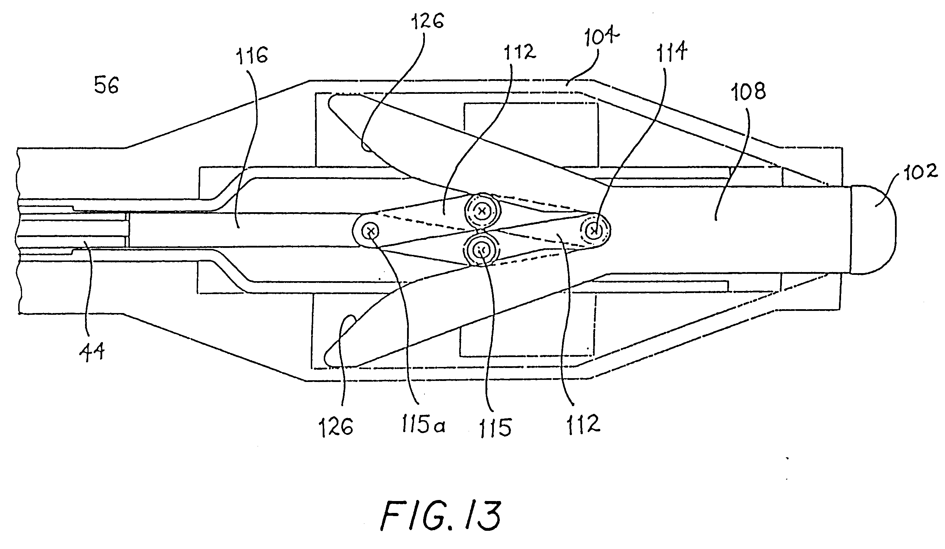

In use, push button 102 is urged distally towards housing 103 so that camming surfaces 126 engage movable pivot posts 115. As linkage structure 110 collapses to the position shown in Fig. 13, movable pivot point 115A urges movable rod forwardly through retaining mechanism 101 to move fastener driver 56 and cartridge frame 44 distally to selectively position the jaw mechanism. When push button 102 is in the position shown in Fig. 13, linkage structure 110 is fully collapsed as shown and movable rod 116 is frictionally secured by clamp member 122.

As seen in Fig. 11, a handle locking mechanism 130 may also be provided. To fire the device to drive fasteners through tissue positioned in jaw mechanism 118, locking mechanism 130 is pivoted away from actuating handle 114 and the fasteners are driven through the tissue in the manner described above. To return instrument 100 to the position shown in Fig. 11, release knob 106 is moved in the direction of arrow H so that clamp member 122 is pivoted about shoulder 120. When clamp member 122 reaches a substantially vertical position perpendicular to movable rod 116, the frictional engagement between the central bore 128 and the movable rod 116 is released, and movable rod 116 returns to the position shown in Fig. 11 due to a biasing spring which is not shown. Release knob 106 is then let go of, and biasing spring 124 returns clamp member 122 to the position shown in Fig. 11. Linkage structure 110 returns to the position shown in Fig. 12.

As described above in connection with linkage structure 42, movement of linkage structure 110 provides for a two-stage approximation of the jaw mechanism, providing for a large approximation (about 80% of the total distance) of the jaw distance for movement of the first 50% of the slider mechanism 108. The remaining 50% of the movement of the slider mechanism 108 moves the jaw mechanism 18 its remaining 20% of distance, providing for fine adjustment.

The adjustable closure mechanism can also be used in other instruments to close the distance between the movable jaw member and stationary jaw member at the stapling or fastening end of the instrument or between two movable jaw members. That is the jaw mechanism may be of the type, wherein one jaw moves toward and away from the other; however, the present invention is also applicable for use with devices of alternative types, i.e., where both jaws move toward and away from each other. The surgical instrument may be of the type which applies metal staples or two part fasteners of the bioabsorbable type.

The surgical stapling or fastening instrument employing the adjustable closure mechanism described above is a device which may be operated with one hand to effect the closure motion of the jaw members of the instrument followed by activation of the trigger mechanism to fire the staples or fasteners into the tissue.

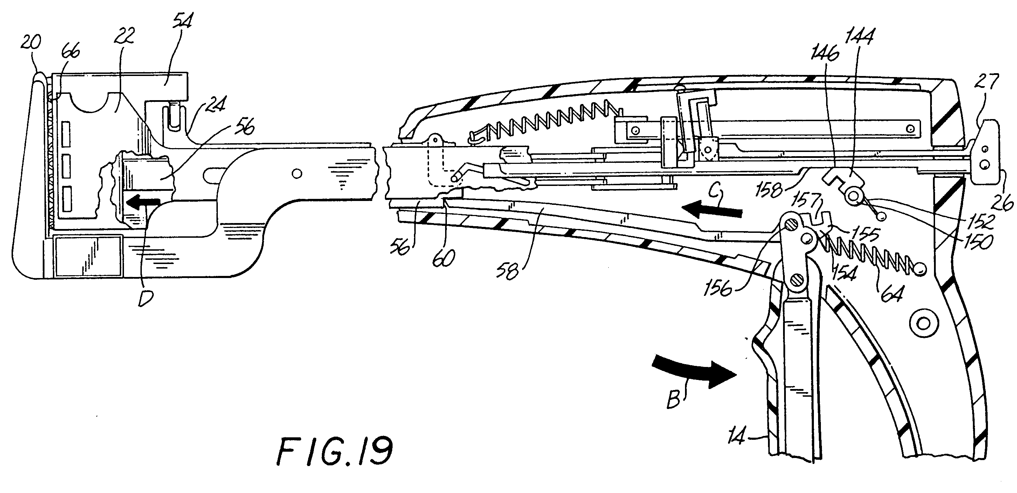

Referring to Figs. 16-19, in operation, the surgical fastening instrument 140 operates substantially similarly to the instrument 10 shown in Figs. 2-6.

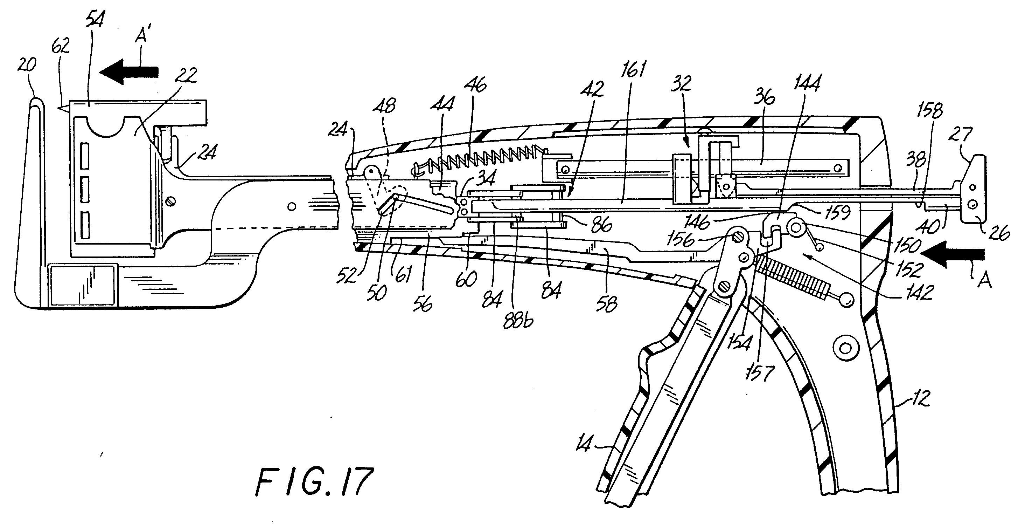

However, in the retracted position (Fig. 16), advancing mechanism 28 is in its proximalmost position and cam member 144 is pivoted into engagement with hook member 154, by surface 161 of slide mechanism 40. As the slide mechanism 40 is moved distally to clamp tissue (Fig. 17), camming surface 159 of groove 158 approaches cam member 144. At this stage, cam member 144 is still positioned in notch 157 so that handle 14 can't be fired.

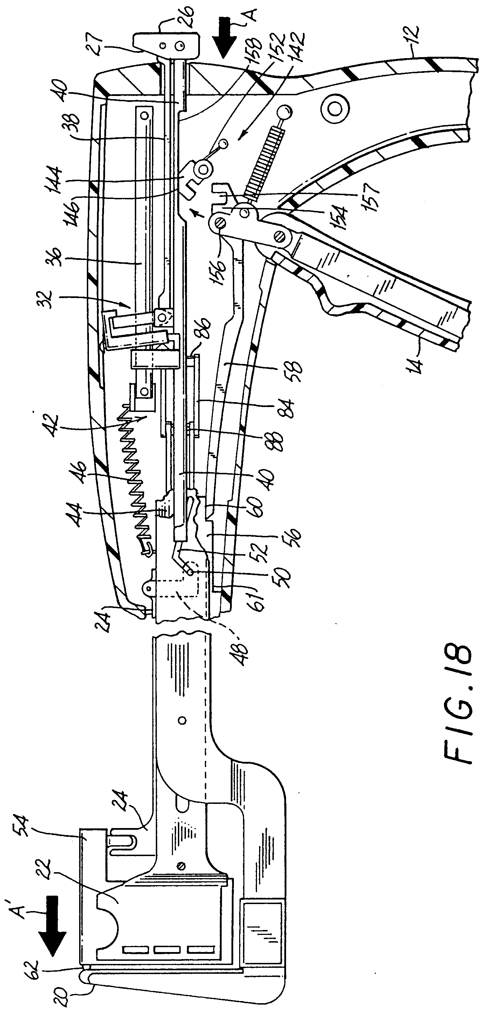

As shown in Fig. 18, once the cartridge jaw 22 has been moved a sufficient distance towards anvil jaw 20, cam member 144 is permitted to pivot into groove 158 thus disengaging arcuate portion 146 from notch 157 in coupling arm 58. Thus, there are a plurality of positions, (e.g. Figs. 18-19), in which the handle 14 of the apparatus can be fired at will by the surgeon.

The claims which follow below include embodiments of the invention additional to those described in detail above.

## Claims

1. An apparatus (10; 100; 140) for applying surgical fasteners to body tissue, comprising:
a frame portion (21);
a handle mechanism (14) depending from said frame portion (21), and coupled to means (56) for driving said fasteners into tissue responsive to movement of said handle mechanism (14);
tissue gripping means including a first jaw member (22) having a plurality of fasteners positioned thereon, and a second jaw member (20), said tissue gripping means positioned at one end of said frame portion (21);
means (40) operable independently of the handle mechanism (14) for advancing one (22) of said jaw members towards said other jaw member to selectively position said one jaw member; and
means (58, 60, 144, 155) for preventing driving of said fasteners, characterised by:
said preventing means being self-deactivating when said first jaw member is positioned a predetermined distance from said second jaw member; and said preventing means being positioned within said frame portion (21).

2. An apparatus according to claim 1, wherein said preventing means prevents movement of said handle mechanism.

3. An apparatus according to claim 2, wherein said preventing means comprises locking means (144) biased into an engaged position by at least a portion of said advancing means (40) to prevent movement of said handle mechanism (14).

4. An apparatus according to claim 3, wherein said advancing means (40), includes a clearance portion (158) for allowing said locking means (144) to disengage said handle mechanism.

5. An apparatus according to claim 4, wherein said locking means (144) is pivotable from an engaged position to a disengaged position in response to movement of said advancing means (40).

6. An apparatus according to claim 3, 4 or 5, wherein said locking means comprises a pawl member (144) which is engageable with a notch (157) in said handle mechanism (14).

7. An apparatus according to claim 6, wherein said locking means comprises a spring biased hook member (144) which is engagable with a corresponding notch (157) in said handle mechanism (14).

8. An apparatus according to any preceding claim, wherein said preventing means prevents movement of said driving means (56).

9. An apparatus according to claim 8, wherein said preventing means includes means (58) for engaging said driving means (56), said engaging means being moved to an engaged position upon advancement of said first jaw member towards said second jaw member.

10. An apparatus according to claim 9, wherein said driving means (56) includes a cam surface (61) over which said engaging means (58) rides, said cam surface having a first surface which contacts said engaging means to hold said engaging means in a disengaged position and to prevent movement of said driving means, and a notch (60) to engage said engaging means to allow movement of said driving means (56).

11. An apparatus according to any one of the preceding claims, wherein said advancing means (40) is linearly actuable.

12. An apparatus according to any one of the preceding claims, further comprising releasable means (32) for retaining said means (40) for advancing along a linear path of travel to selectively position said one (22) of the jaw members.

## Patentansprüche

1. Eine Vorrichtung (10; 100; 140), um Operationsbefestiger an Körpergewebe anzubringen, umfassend:
einen Rahmenabschnitt (21);
einen Griffmechanismus (40), der am Rahmenabschnitt (21) angebracht ist und mit einer Vorrichtung (56) gekoppelt ist, um die Befestiger in Reaktion auf eine Bewegung des Griffmechanismus (14) in das Gewebe hineinzutreiben;
eine Gewebegreifvorrichtung einschließlich eines ersten Backenbauteils (22) mit einer Vielzahl von darauf angeordneten Befestigern, und eines zweiten Backenbauteils (20), wobei die Gewebegreifvorrichtung an einem Ende des Rahmenabschnitts (21) angeordnet ist;
eine Vorrichtung (40), die unabhängig von dem Griffmechanismus (14) bedient werden kann, um eines (22) der Backenbauteile auf das andere Backenbauteil zuzubewegen, um selektiv das eine Backenbauteil zu positionieren; und
eine Vorrichtung (58, 60, 144, 155), um ein Auslösen der Befestiger zu verhindern, dadurch gekennzeichnet, daß die Verhinderungsvorrichtung selbst-deaktivierend ist, wenn das erste Backenbauteil in einem bestimmten Abstand vom zweiten Backenbauteil angeordnet ist; und die Verhinderungsvorrichtung innerhalb des Rahmenabschnitts (21) angeordnet ist.

2. Eine Vorrichtung nach Anspruch 1, dadurch gekennzeichnet, daß die Verhinderungsvorrichtung eine Bewegung des Griffmechanismus verhindert.

3. Eine Vorrichtung nach Anspruch 2, dadurch gekennzeichnet, daß die Verhinderungsvorrichtung eine Schließvorrichtung (144) einschließt, die durch zumindest einen Abschnitt der Vorschubvorrichtung (40) in eine eingekuppelte Position vorgespannt wird, um eine Bewegung des Griffmechanismus (14) zu verhindern.

4. Eine Vorrichtung nach Anspruch 3, dadurch gekennzeichnet, daß die Vorschubvorrichtung (40) einen Abstandsteil (158) einschließt, um zu erlauben, daß die Schließvorrichtung (144) aus dem Griffmechanismus auskuppeln kann.

5. Eine Vorrichtung nach Anspruch 4, dadurch gekennzeichnet, daß der Schließmechanismus (144) in Reaktion auf eine Bewegung der Vorschubvorrichtung (40) aus einer eingekuppelten Position in eine ausgekuppelte Position drehbar ist.

6. Eine Vorrichtung nach Anspruch 3, 4 oder 5, dadurch gekennzeichnet, daß die Schließvorrichtung ein Klinkbauteil (144) einschließt, das in eine Nut (157) in dem Griffmechanismus (14) eingekuppelt werden kann.

7. Eine Vorrichtung nach Anspruch 6, dadurch gekennzeichnet, daß der Schließmechanismus ein durch eine Feder vorgespanntes Hakenbauteil (144) einschließt, das mit einer entsprechenden Nut (157) im Griffmechanismus (14) einkuppeln kann.

8. Eine Vorrichtung nach einem der vorhergehenden Ansprüche, dadurch gekennzeichnet, daß die Verhinderungsvorrichtung eine Bewegung der Antriebsvorrichtung (56) verhindert.

9. Eine Vorrichtung nach Anspruch 8, dadurch gekennzeichnet, daß die Verhinderungsvorrichtung eine Vorrichtung (58) einschließt, um die Treibervorrichtung (56) einzukuppeln, wobei die Kupplungsvorrichtung aufgrund einer Vorwärtsbewegung des ersten Backenbauteils auf das zweite Backenbauteil zu in eine eingekuppelte Position bewegt wird.

10. Eine Vorrichtung nach Anspruch 9, dadurch gekennzeichnet, daß die Treibervorrichtung (56) eine Nockenoberfläche (61) einschließt, über die die Einkuppelvorrichtung (58) gleitet, wobei die Nockenoberfläche eine erste Oberfläche aufweist, die die Einkuppelvorrichtung kontaktiert, um die Einkuppelvorrichtung in einer ausgekuppelten Position zu halten, und um eine Bewegung der Treibervorrichtung zu verhindern, und eine Nut (60), um die Einkuppelvorrichtung einzukuppeln, um eine Bewegung der Treibervorrichtung (56) zu erlauben.

11. Eine Vorrichtung nach einem der vorhergehenden Ansprüche, dadurch gekennzeichnet, daß die Vorschubvorrichtung (40) linear einstellbar ist.

12. Eine Vorrichtung nach einem der vorhergehenden Ansprüche, weiter eine auslösbare Vorrichtung (32) umfassend, um die Vorrichtung (40) zurückzuhalten, um entlang eines linearen Bewegungspfades sich vorwärts zu bewegen, um selektiv das eine (22) der Backenbauteile zu positionieren.

## Revendications

1. Un appareil (10 ; 100 ; 140) pour appliquer des attaches chirurgicales à du tissu corporel comprenant :
une portion de châssis (21) ;
un mécanisme de poignée (14) s'étendant depuis ladite portion de châssis (21) et accouplé à un moyen (56) pour entraîner lesdites attaches dans le tissu en réponse à un déplacement dudit mécanisme de poignée (14) ;
des moyens de préhension de tissu incluant un premier élément de mâchoire (22) ayant une pluralité d'attaches positionnées sur celui-ci, et un deuxième élément de mâchoire (20), ledit moyen de préhension de tissu étant positionné à une extrémité de ladite portion de châssis (21) ;
un moyen (40) actionnable indépendamment du mécanisme de poignée (14) pour faire avancer l'un (22) desdits éléments de mâchoire vers ledit autre élément de mâchoire pour positionner sélectivement ledit élément de mâchoire précité ; et
des moyens (58, 60, 144, 155) pour empêcher l'entraînement desdites attaches, caractérisé par :
lesdits moyens d'empêchement étant à auto-désactivation lorsque ledit premier élément de mâchoire est positionné à une distance prédéterminée dudit deuxième élément de mâchoire ; et lesdits moyens d'empêchement étant positionnés dans ladite portion de châssis (21).

2. Appareil selon la revendication 1, où lesdits moyens d'empêchement empêchent le déplacement dudit mécanisme de poignée.

3. Appareil selon la revendication 2, où lesdits moyens d'empêchement comprennent des moyens de verrouillage (144) sollicités en une position en prise par au moins une partie dudit moyen d'avancement (40) pour empêcher le déplacement dudit mécanisme de poignée (14).

4. Appareil selon la revendication 3, où ledit moyen d'avancement (40) comporte une portion de dégagement (158) pour permettre que ledit moyen de verrouillage (144) se dégage dudit mécanisme de poignée.

5. Appareil selon la revendication 4, où ledit moyen de verrouillage (144) peut être amené à pivoter d'une position en prise en une position hors prise en réponse à un déplacement dudit moyen d'avancement (40).

6. Appareil selon la revendication 3, 4 ou 5, où ledit moyen de verrouillage comprend un élément de crochet (144) qui peut être engagé dans une encoche (157) dans ledit mécanisme de poignée (14).

7. Appareil selon la revendication 6, où ledit moyen de verrouillage comprend un élément de crochet (144) sollicité par ressort qui peut s'engager dans une encoche correspondante (157) dans ledit mécanisme de poignée (14).

8. Appareil selon l'une des revendications précédentes, où ledit moyen d'empêchement empêche le déplacement dudit moyen d'entraînement (56).

9. Appareil selon la revendication 8, où ledit moyen d'empêchement comporte un moyen (58) pour l'engagement dudit moyen d'entraînement (56), ledit moyen d'engagement étant amené en une position engagée lors de l'avance dudit premier élément de mâchoire vers ledit deuxième élément de mâchoire.

10. Appareil selon la revendication 9, où ledit moyen d'entraînement (56) comporte une surface de came (61) sur laquelle se déplace ledit moyen d'engagement (58), ladite surface de came ayant une première surface qui vient en contact avec ledit moyen d'engagement pour tenir ledit moyen d'engagement dans une position hors prise et pour empêcher un déplacement dudit moyen d'entraînement, et une encoche (60) pour engager ledit moyen d'engagement pour permettre le déplacement dudit moyen d'entraînement (56).

11. Appareil selon l'une des revendications précédentes, où ledit moyen d'avancement (40) est actionnable linéairement.

12. Appareil selon l'une des revendications précédentes, comprenant en outre un moyen de relâchement (32) pour retenir ledit moyen (40) pour avancer le long d'un trajet de déplacement linéaire afin de positionner sélectivement ledit élément (22) précité des éléments de mâchoire.
